# EUROPEAN PATENT SPECIFICATION

(11) **EP 2 540 337 B1**
(45) Date of publication and mention of the grant of the patent: **03.04.2019**
(21) Application number: 11747468.4
(22) Date of filing: 24.02.2011
(51) Int. Cl.: A61M 37/00

(54) **MICRO-NEEDLE DEVICE AND PREPARATION METHOD**
MIKRONADELVORRICHTUNG UND HERSTELLUNGSVERFAHREN DAFÜR
DISPOSITIF À MICRO-AIGUILLES ET SON PROCÉDÉ DE PRÉPARATION

(30) Priority: 24.02.2010 JP 2010039318
(43) Date of publication of application: 02.01.2013
(73) Proprietor: Hisamitsu Pharmaceutical Co., Inc., Tosu-shi, Saga 841-0017 (JP)
(72) Inventor: MATSUDO Toshiyuki, Tsukuba-shi Ibaraki 305-0856 (JP); NISHIMURA Shinpei, Tsukuba-shi Ibaraki 305-0856 (JP); TOKUMOTO Seiji, Tsukuba-shi Ibaraki 305-0856 (JP); MORIMOTO Kumi, Tsukuba-shi Ibaraki 305-0856 (JP)
(74) Representative: Grünecker Patent- und Rechtsanwälte PartG mbB
(86) International application number: PCT/JP2011/054177
(87) International publication number: WO 2011/105508

(56) References cited:
- EP-A1- 2 441 437
- WO-A1-2009/051147
- WO-A2-2006/138719
- JP-A- 2004 504 120
- JP-B2- 2 926 742

## Description

### Technical Field

The present invention relates to a micro-needle device and a method for preparing the device.

### Background Art

Conventionally, a micro-needle device has been known as a device for improving transdermal absorption of a pharmaceutical drug. Micro-needles provided on the micro-needle device are intended to pierce the stratum corneum as the outermost skin layer, micro-needles having various sizes and various shapes have been developed, and such a micro-needle is expected as a noninvasive administration method (for example, Patent Literature 1).

Various methods have been developed for applying pharmaceutical drugs using a micro-needle device. Examples of the known method include coating a micro-needle surface with a pharmaceutical drug, providing a groove or a hollow portion in a micro-needle for penetration of a pharmaceutical drug or a biogenic substance, and mixing a pharmaceutical drug into a micro-needle itself. It is disclosed that, at this time, a substance to be combined with a pharmaceutical drug for the coating preferably contains sugars, in particular, stabilizing sugars that form a glass (amorphous solid), such as lactose, raffinose, trehalose, and sucrose (Patent Literature 2).

Patent Literature 3 also discloses an apparatus and a method for transdermal delivery of a biologically active drug including a delivery system having a microprojection member. In an embodiment of the apparatus and the method, it is disclosed that a biocompatible coating formulation applied to the microprojection member includes at least one nonaqueous solvent, for example, ethanol, isopropanol, methanol, propanol, butanol, propylene glycol, dimethyl sulfoxide, glycerin, N,N-dimethylformamide, and polyethylene glycol 400 and the nonaqueous solvent is preferably present in the coating formulation in the range of about 1% by weight to 50% by weight of the coating formulation. It is also disclosed that the coating formulation has a viscosity of 3 to about 500 centipoise (cps). Patent Literature 4 provides a microneedle device for insertion of a drug into a biological tissue that includes at least one microneedle and a coating on at least a portion of the surface of the at least one microneedle, the coating comprising at least one drug and a viscosity enhancer.

### Citation List

[Patent Literature 1] Japanese Unexamined Patent Application Publication No. 2001-506904
[Patent Literature 2] Japanese Unexamined Patent Application Publication No. 2004-504120
[Patent Literature 3] Japanese Unexamined Patent Application Publication No. 2007-536988 [Patent Literature 4] WO 2006/138719 A2

### Summary of Invention

### Technical Problem

However, in the production of a micro-needle device using a composition (physiologically active composition) containing a physiologically active substance and a solvent as disclosed in Patent Literatures 1 to 4, it is revealed that a preparation method that includes storing the physiologically active composition in a container from which a solvent can volatilize and then depositing the physiologically active composition onto the micro-needles raises a problem when the physiologically active composition is deposited onto a number of micro-needle arrays (onto micro-needles). In other words, when micro-needle devices are continuously produced by such a method, it is revealed that there is a problem in which the amount of the physiologically active composition applied onto the micro-needles largely varies to interfere with the production of the micro-needle device having a stable coating amount. The variation in the amount of a pharmaceutical drug between micro-needle devices produced is not preferred from the medical (therapeutic) viewpoint as well as the economic viewpoint especially when a physiologically active substance to be used has strong effect or is expensive.

In view of the above circumstances, an object of the present invention is to provide a method according to claim 3 for preparing a micro-needle device that reduces the variation of the deposition amount of a physiologically active substance deposited on micro-needles to a level sufficient for practical use even when the continuous preparation method as described above using a mask plate is employed. Another object of the present invention is to provide a micro-needle device according to claim 1 that can be obtained by the preparation method. Another object of the present invention is to provide a method according to claim 6 of stabilizing a deposition amount of a physiologically active composition onto a micro-needle device.

### Solution to Problem

The present invention provides a method for preparing a micro-needle device that includes the step of depositing a physiologically active composition containing a physiologically active substance and a solvent capable of dispersing or dissolving the physiologically active substance onto a micro-needle. In the method, at least one polyhydric alcohol selected from glycerin, ethylene glycol, propylene glycol and 1,3-butylene glycol is used, and water is not used, as the solvent. In the method, at least one compound selected from polyethylene glycol, chondroitin sulfate, dextran, croscarmellose sodium and a metal chloride is used.

The method for preparing a micro-needle device having such a constitution leads to small variation in the viscosity of the physiologically active composition with time in the production even when the production is continuously carried out, thereby capable of stably obtaining a micro-needle device having micro-needles on which the physiologically active composition containing a physiologically active substance is deposited in a uniform (less varied) amount. The reason why such a preparation method can produce the micro-needle device having a physiologically active substance in a stable deposition amount is supposed to be because the solvent without water largely contributes.

The deposition amount of the physiologically active substance is remarkably stabilized when a preparation method is applied in which a container is a mask plate having an opening, the opening is filled with the physiologically active composition, then the micro-needle is inserted into the opening and is pulled out of the opening to deposit the physiologically active composition onto the micro-needle.

As described above, by the preparation method, the deposition amount of the physiologically active substance is remarkably stabilized. Hence, as the mask plate filled with the physiologically active composition, one micro-needle is pulled out of a mask plate and then the same mask plate may be reused with respect to another micro-needle. In addition to the method of using the mask plate, for example, a method that includes storing a physiologically active composition containing a physiologically active substance and a solvent capable of dispersing or dissolving the physiologically active substance in a liquid pool having an open upper end and transferring the physiologically active composition, for example, close to the micro-needle using a pump or the like for spray-coating can be exemplified as another method.

It is preferable that a mass ratio of the physiologically active substance and the polyhydric alcohol is 20:80 to 80:20 in the physiologically active composition, and it is preferable that the physiologically active composition have a viscosity of 0.600 to 45.000 Pa.s (600 to 45000 cps) at room temperature (25°C). By adopting such a condition, the physiologically active substance is surely contained in the physiologically active composition deposited on the micro-needle with ease depending on the amount of the physiologically active substance in the physiologically active composition. Therefore, a micro-needle device having high administration efficiency of the physiologically active substance can be obtained.

The preparation method can be embodied as a method of stabilizing a deposition amount of a physiologically active composition. The method includes the steps of storing a physiologically active composition containing a physiologically active substance and a solvent capable of dispersing or dissolving the physiologically active substance in a container from which the solvent is capable of volatilizing, and then depositing the physiologically active composition onto a micro-needle to produce a micro-needle device. In the method, at least one polyhydric alcohol selected from glycerin, ethylene glycol, propylene glycol, and 1,3-butylene glycol is used as the solvent and water is not used. In the method, at least one compound selected from polyethylene glycol, chondroitin sulfate, dextran, croscarmellose sodium and a metal chloride is used.

The present invention also provides a micro-needle device that includes a substrate, a micro-needle provided on the substrate, and a physiologically active composition deposited on the micro-needle and/or the substrate. In the micro-needle device, the physiologically active composition contains: at least one polyhydric alcohol selected from glycerin, ethylene glycol, propylene glycol and 1,3-butylene glycol; at least one compound selected from polyethylene glycol, chondroitin sulfate, dextran, croscarmellose sodium and a metal chloride; and a physiologically active substance, and contains substantially no water.

Here, in the physiologically active composition deposited on the micro-needle and/or the substrate, "contains substantially no water" means not containing water in an amount more than the water content due to moisture absorption from air after the deposition of the physiologically active composition. The water content is typically 7% by mass or less, preferably 5% by mass or less, and more preferably 3% by mass or less, based on the total amount of the deposited physiologically active composition.

In the micro-needle device, it is necessary that the physiologically active composition deposited on the micro-needle and/or the substrate further contains at least one compound selected from polyethylene glycol, chondroitin sulfate, dextran, croscarmellose sodium, and magnesium chloride.

A micro-needle device having such a constitution can improve the viscosity of the physiologically active composition to highly control the height of the physiologically active composition deposited on the micro-needle and/or the substrate, and the amount of the physiologically active substance.

It is preferable that the physiologically active composition deposited on the micro-needle and/or the substrate be dried and fixed after the application onto the micro-needle and/or the substrate.

### Advantageous Effects of Invention

The present invention provides a method for preparing a micro-needle device that reduces the variation of the deposition amount of a physiologically active substance deposited onto a micro-needle to a level sufficient for practical use even when a continuous preparation method using a mask plate is employed, and provides a micro-needle device that can be obtained by the preparation method.

### Brief Description of Drawings

Fig. 1 is a perspective view showing an example of a micro-needle device according to an embodiment of the present invention.
Fig. 2 is a sectional view taken along the line II-II in Fig. 1.
Figs. 3(a) to 3(c) are views showing an example of a method for preparing the micro-needle device.
Fig. 4 is a graph showing time course of the amount of a physiologically active substance in a physiologically active composition deposited on micro-needles when a filling and deposition process of the physiologically active composition is repeated to produce micro-needle devices.
Fig. 5 is a graph showing time course of blood lixisenatide concentrations when lixisenatide is administered with the micro-needle device and is subcutaneously administered.
Fig. 6 is a graph showing time course of blood β-interferon concentrations when β-interferon is administered with the micro-needle device and is subcutaneously administered.

### Description of Embodiments

Hereinafter, preferred embodiments will be described with reference to drawings. In the description of the drawings, identical elements are represented by the same reference numbers and redundant description will be omitted. In the drawings, some elements are shown on larger scales for easy understanding and the elements are not necessarily shown in proportion to those in the descriptions.

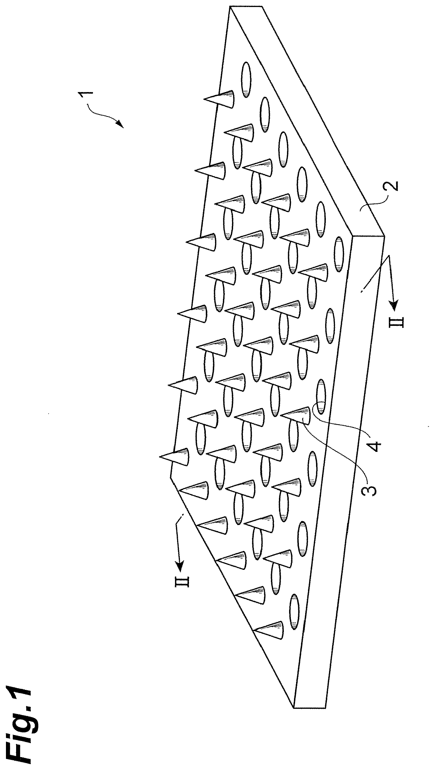

Fig. 1 is a perspective view showing an embodiment of a micro-needle device of the present invention. As shown in Fig. 1, this micro-needle device 1 includes a micro-needle substrate 2, and a plurality of micro-needles 3 that are two-dimensionally arranged on the micro-needle substrate 2.

The micro-needle substrate 2 is a base for supporting the micro-needles 3. The shape of the micro-needle substrate 2 is not specifically limited and, for example, the micro-needle substrate 2 may include a plurality of through-holes 4 so as to be two-dimensionally arranged. The micro-needles 3 and the through-holes 4 are alternately arranged in a diagonal direction of the micro-needle substrate 2. Through the through-holes 4, a physiologically active composition can be administered from the back face of the micro-needle substrate 2. Alternatively, a substrate without such a through-hole may be used. The micro-needle substrate 2 has an area of 0.5 to 10 cm², preferably 1 to 5 cm², and more preferably 1 to 3 cm². Several of the micro-needle substrates 2 may be connected to form a substrate having a desired size.

The micro-needle 3 has a minute structure and preferably has a height (length) of 50 to 600 µm. The reason why the micro-needle 3 has a length of 50 µm or more is to ensure the transdermal administration of a physiologically active substance, while the reason why the micro-needle 3 has a length of 600 µm or less is to avoid the contact between the micro-needle and nerves to reduce the possibility of pain and to reduce the possibility of bleeding. The micro-needle 3 having a length of 500 µm or less enables efficient administration of a physiologically active substance in an amount to be released inside the skin and, depending on a condition, enables the administration without piercing the skin. The micro-needle 3 particularly preferably has a length of 300 to 500 µm.

The micro-needle means a projecting structure including, in a broad sense, a needle shape or a structure containing a needle shape. However, the micro-needle is not limited to a structure having a needle shape with a sharp tip but includes a structure without a sharp tip. The micro-needle 3 having a conical structure has a base diameter of about 50 to 200 µm. The micro-needle 3 has a conical shape in the present embodiment, but micro-needles having a polygonal pyramid shape such as a square pyramid and having other shapes may also be used.

The micro-needles 3 are typically spaced for arrangement so as to provide a density of about one to ten needles per millimeter (mm) in a row of the needles. Commonly, adjacent rows are spaced apart from each other by a distance substantially equal to the space between the needles in a raw, and the needle density is 100 to 10,000 needles per cm². A device having a needle density of 100 needles or more enables efficient piercing of the skin. In contrast, a device having a needle density of more than 10,000 needles is difficult to maintain the strength of the micro-needles 3. The density of the micro-needles 3 is preferably 200 to 5,000 needles, more preferably 300 to 2,000 needles, and most preferably 400 to 850 needles.

Examples of a material of the micro-needle substrate 2 or the micro-needle 3 include silicon, silicon dioxide, ceramics, metals (such as stainless steel, titanium, nickel, molybdenum, chromium, and cobalt), and synthetic or natural resin materials. In consideration of the antigenicity of the micro-needle and the unit price of the material, particularly preferred materials are synthetic or natural resin materials including a biodegradable polymer such as polylactic acid, polyglycolide, polylactic acid-co-polyglycolide, pullulan, capronolactone, polyurethane and polyanhydride, and a nondegradable polymer such as polycarbonate, polymethacrylic acid, ethylene vinyl acetate, polytetrafluoroethylene and polyoxymethylene. Additional preferred examples include polysaccharides such as hyaluronic acid, sodium hyaluronate, pullulan, dextran, dextrin, and chondroitin sulfate.

Examples of a preparation method of the micro-needle substrate 2 or the micro-needle 3 include a wet etching process or a dry etching process using a silicon substrate, precision machining using metals or resins (such as an electric discharge method, laser processing, dicing processing, a hot embossing process, and injection mold processing), and machinery cutting. By such a processing method, a needle part and a supporting part are molded as a single-piece. Examples of a method for hollowing the needle part include a secondary processing such as laser processing after the preparation of the needle part.

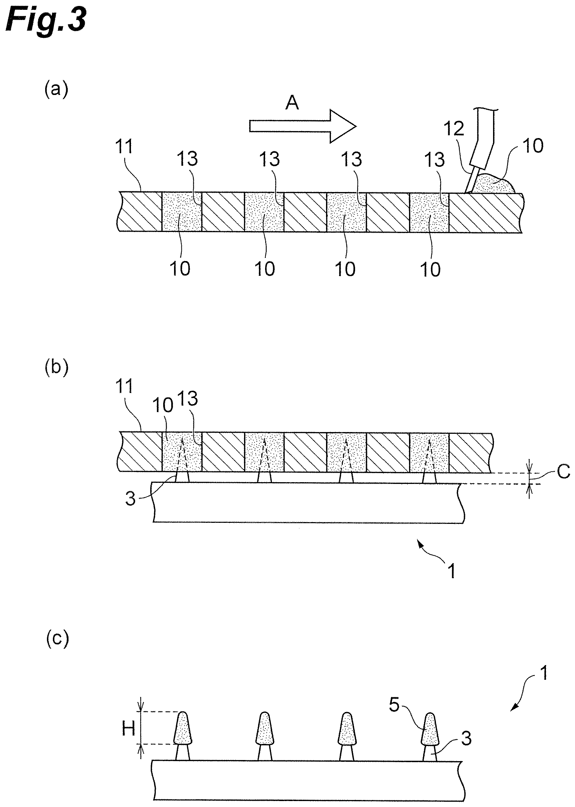

Figs. 3(a) to 3(c) are views showing an example of the preparation method of the micro-needle device 1. In the method, first, as shown in Fig. 3(a), a physiologically active composition 10 is swept on a mask plate 11 with a spatula 12 in the direction of an arrow A. By this operation, the physiologically active composition is filled into openings 13. Subsequently, as shown in Fig. 3(b), the micro-needles 3 are inserted into the openings 13 of the mask plate 11. Then, as shown in Fig. 3(c), the micro-needles 3 are pulled out of the openings 13 of the mask plate 11. By this operation, the physiologically active composition 10 is deposited (applied in this case) onto the micro-needles 3. Then, the physiologically active composition on the micro-needles is dried by a known manner such as air-drying, vacuum drying, and freeze-drying or by combination of them. By this operation, the solid physiologically active composition 10 is fixed onto the micro-needles 3 as a physiologically active composition 5 deposited on the micro-needles 3. In this manner, the micro-needle device is produced. The term "fixed" means a state in which the physiologically active composition keeps being almost uniformly deposited on an object.

The height H of the physiologically active composition deposited on the micro-needles 3 (on the micro-needles 3 and/or on the substrate) can be controlled by a clearance (gap) C shown in Fig. 3(b). The clearance C is defined as a distance from the basal surface of the micro-needle to the mask surface (substrate thickness is not involved), and is designed depending on a tension of the mask plate 11 and the length of the micro-needle 3. The clearance C preferably has a distance ranging from 0 to 500 µm. The clearance C having a distance of 0 means that the physiologically active composition is deposited onto the whole micro-needle 3. The height H of the physiologically active composition 5 deposited on the micro-needles 3 varies depending on the height h of the micro-needle 3. However, the height H may be 0 to 500 µm and is typically 10 to 500 µm and preferably about 30 to 300 µm.

The physiologically active composition 5 deposited on the micro-needles 3 has a thickness of less than 50 µm, preferably less than 40 µm, and more preferably 1 to 30 µm. Generally, the thickness of the physiologically active composition deposited on the micro-needles 3 is an average thickness as measured over the surface of the micro-needles 3 after drying. The thickness of the physiologically active composition deposited on the micro-needles 3 can generally be increased by multiple applications of the physiologically active composition, that is, by repeating the deposition process of the physiologically active composition onto the micro-needles 3.

When the physiologically active composition is deposited onto the micro-needles 3 and/or the substrate 2, an installation environment of an apparatus is preferably controlled at a constant temperature and a constant humidity. The environment may be, as necessary, filled with a "solvent including at least one polyhydric alcohol selected from the group consisting of glycerin, ethylene glycol, propylene glycol and 1,3-butylene glycol" as a component (B) described later that is used in the physiologically active composition. Such a condition can suppress the volatilization of the solvent in the physiologically active composition as much as possible.

The physiologically active composition contains the "physiologically active substance" (A) and a "solvent including at least one polyhydric alcohol selected from the group consisting of glycerin, ethylene glycol, propylene glycol and 1,3-butylene glycol" (B). The physiologically active composition further contains at least one compound selected from polyethylene glycol, chondroitin sulfate, dextran, croscarmellose sodium and a metal chloride. The physiologically active composition does not substantially contain water. Here, in the physiologically active composition, "not substantially containing water" means that the physiologically active composition does not contain water in an amount more than the water content due to moisture absorption from air. The water content is typically 20% by mass or less, preferably 10% by mass or less, and more preferably 5% by mass or less, based on the total amount of the physiologically active composition. The component (B) is preferably a "solvent including only at least one polyhydric alcohol selected from the group consisting of glycerin, ethylene glycol, propylene glycol and 1,3-butylene glycol alone." The "physiologically active substance" is a substance having any effect on a living body and includes low-molecular compounds, peptides, proteins, and derivatives of them. The "solvent" means a compound that can disperse or dissolve the physiologically active substance. Examples of the physiologically active substance (drug) (A) include, but are not necessarily limited to, polymer compounds such as peptides, proteins, DNAs, and RNAs. The physiologically active substance (drug) (A) may be, for example, vaccines, low-molecular peptides, sugars, and nucleic acids as long as the molecular weight is about 1,000. Examples of the physiologically active substance include lixisenatide, naltrexone, cetrorelix acetate, taltirelin, nafarelin acetate, prostaglandin Al, alprostadil, α-interferon, β-interferon for multiple sclerosis, erythropoietin, follitropin β, follitropin α, G-CSF, GM-CSF, human chorionic gonadotropin, luteinizing hormone, calcitonin salmon, glucagon, GNRH antagonists, insulin, human growth hormone, filgrastim, heparin, low molecular heparin, somatropin, incretin, and GLP-1 derivatives. Examples of the vaccines include a Japanese encephalitis vaccine, a rotavirus vaccine, an Alzheimer's disease vaccine, an arteriosclerosis vaccine, a cancer vaccine, a nicotine vaccine, a diphtheria vaccine, a tetanus vaccine, a pertussis vaccine, a Lyme disease vaccine, a rabies vaccine, a Diplococcus pneumoniae vaccine, a yellow fever vaccine, a cholera vaccine, a vaccinia vaccine, a tuberculosis vaccine, a rubella vaccine, a measles vaccine, a mumps vaccine, a botulinum vaccine, a herpes virus vaccine, other DNA vaccines, and a hepatitis B vaccine.

Additional examples of the physiologically active substance include hypnotics and sedatives (such as flurazepam hydrochloride, rilmazafone hydrochloride, phenobarbital, and amobarbital), antipyretic analgesic anti-inflammatory drugs (such as butorphanol tartrate, perisoxal citrate, acetaminophen, mefenamic acid, diclofenac sodium, aspirin, alclofenac, ketoprofen, flurbiprofen, naproxen, piroxicam, pentazocine, indomethacin, glycol salicylate, aminopyrine, and loxoprofen), steroidal anti-inflammatory drugs (such as hydrocortisone, prednisolone, dexamethasone, and betamethasone), stimulant drugs (such as methamphetamine hydrochloride and methylphenidate hydrochloride), antipsychotic drugs (such as imiprane hydrochloride, diazepam, sertraline hydrochloride, fluvoxamine maleate, paroxetine hydrochloride, citalopram hydrobromide, fluoxetine hydrochloride, alprazolam, haloperidol, clomipramine, amitriptyline, desipramine, amoxapine, maprotiline, mianserin, setiptiline, trazadone, lofepramine, milnacipran, duloxetine, venlafaxine, chlorpromazine hydrochloride, thioridazine, diazepam, meprobamate, and etizolam), hormones (such as estradiol, estriol, progesterone, norethisterone acetate, methelonone acetate, and testosterone), local anesthetics (such as lidocaine hydrochloride, procaine hydrochloride, tetracaine hydrochloride, dibucaine hydrochloride, and propitocaine hydrochloride), agents acting upon the urinary organs (such as oxybutynin hydrochloride, tamsulosin hydrochloride, and propiverine hydrochloride), skeletal muscle relaxants (such as tizanidine hydrochloride, eperisone hydrochloride, pridinol mesylate, and suxamethonium hydrochloride), agents acting upon the reproductive organs (ritodrine hydrochloride and meluadrine tartrate), antiepileptic drugs (such as sodium valproate, clonazepam, and carbamazepine), autonomic drugs (such as carpronium chloride, neostigmine bromide, and bethanechol chloride), antiparkinsonian drugs (such as pergolide mesylate, bromocriptine mesylate, trihexyphenidyl hydrochloride, amantadine hydrochloride, ropinirole hydrochloride, talipexole hydrochloride, cabergoline, droxidopa, piperiden, and selegiline hydrochloride), diuretics (such as hydroflumethiazide and furosemide), respiratory stimulants (such as lobeline hydrochloride, dimorpholamine, and naloxone hydrochloride), antimigraine drugs (such as dihydroergotamine mesylate, sumatriptan, ergotamine tartrate, flunarizine hydrochloride, and cyproheptadine hydrochloride), antihistamines (such as clemastine fumarate, diphenhydramine tannate, chlorpheniramine maleate, diphenylpyraline hydrochloride, and promethazine), bronchodilators (such as tulobuterol hydrochloride, procaterol hydrochloride, salbutamol sulfate, clenbuterol hydrochloride, fenoterol hydrobromide, terbutaline sulfate, isoprenaline sulfate, and formoterol fumarate), cardiotonic agents (such as isoprenaline hydrochloride and dopamine hydrochloride), coronary vasodilators (such as diltiazem hydrochloride, verapamil hydrochloride, isosorbide dinitrate, nitroglycerin, and nicorandil), peripheral vasodilators (such as nicametate citrate and tolazoline hydrochloride), stop smoking aids (such as nicotine), circulatory drug (such as flunarizine hydrochloride, nicardipine hydrochloride, nitrendipine, nisoldipine, felodipine, amlodipine besylate, nifedipine, nilvadipine, manidipine hydrochloride, benidipine hydrochloride, enalapril maleate, democapril hydrochloride, alacepril, imidapril hydrochloride, cilazapril, lisinopril, captopril, trandolapril, perindopril erbumine, atenolol, bisoprolol fumarate, metoprolol tartrate, betaxolol hydrochloride, arotinolol hydrochloride, celiprolol hydrochloride, carvedilol, carteolol hydrochloride, bevantolol hydrochloride, valsartan, candesartan cilexetil, losartan potassium, and clonidine hydrochloride), antiarrhythmic drugs (such as propranolol hydrochloride, alprenolol hydrochloride, procainamide hydrochloride, mexitilene hydrochloride, nadolol, and disopyramide), anti-malignant ulcer agents (such as cyclophosphamide, fluorouracil, degafur, procarbazine hydrochloride, ranimustine, irinotecan hydrochloride, and fluridine), antilipemic agents (such as pravastatin, simvastatin, bezafibrate, and probucol), hypoglycemic agents (glibenclamide, chlorpropamide, tolbutamide, glymidine sodium, glybuzole, and buformin hydrochloride), antiulcer drugs (proglumide, cetraxate hydrochloride, spizofurone, cimetidine, and glycopyrronium bromide), cholagogues (such as ursodesoxycholic acid and osalmid), prokinetic drugs (such as domperidone and cisapride), agents for liver disorder (such as tiopronin), antiallergic agents (such as ketotifen fumarate and azelastine hydrochloride), antivirals (such as aciclovir), antidinics (such as betahistine mesylate and difenidol hydrochloride), antibiotics (such as cefaloridine, cefdinir, cefpodoxime proxetil, cefaclor, clarithromycin, erythromycin, methylerythromycin, kanamycin sulfate, cycloserine, tetracycline, benzylpenicillin potassium, propicillin potassium, cloxacin sodium, ampicillin sodium, bacampicillin hydrochloride, carbenicillin sodium, and chloramphenicol), agents for habitual addiction (such as cyanamide), anorectic agents (such as mazindol), chemotherapeutics (such as isoniasid, ethionamide, and pyrazinamide), blood-clotting agents (ticlopidine hydrochloride and warfarin potassium), anti-Alzheimer agents (such as physostigmine, donepezil hydrochloride, tacrine, arecoline, and xanomeline), serotonin receptor antagonist antiemetics (such as ondansetron hydrochloride, granisetron hydrochloride, ramosetron hydrochloride, and azasetron hydrochloride), antipodagrics (such as colchicine, probenecid, and sulfinpyrazone), and narcotic analgesics (such as fentanyl citrate, morphine sulfate, morphine hydrochloride, codeine phosphate, cocaine hydrochloride, and pethidine hydrochloride).

These drugs may be used alone or in combination of two or more of them and, needless to say, a drug in a form of either an inorganic salt or an organic salt is encompassed as long as the salt is pharmaceutically acceptable. The physiologically active composition contains the physiologically active substance (A) in an amount of 0.1 to 80% by mass, preferably 1 to 70% by mass, and particularly preferably 5 to 60% by mass.

The "solvent including at least one polyhydric alcohol selected from the group consisting of glycerin, ethylene glycol, propylene glycol and 1,3-butylene glycol" (B) has a high boiling point and volatilizes in a small amount during the filling and deposition process. Hence, even when the micro-needle devices are continuously produced, the viscosity change of the physiologically active composition is small. In addition, such a solvent has a high solubility or a high dispersibility with respect to the physiologically active substance. Therefore, such a solvent can achieve the production of a micro-needle device having micro-needles on which the physiologically active composition is deposited in a uniform amount. In the physiologically active composition, the compounding ratio (A:B) of the component (A) and the component (B) is preferably 20:80 to 80:20, more preferably 40:60 to 80:20, and most preferably 50:50 to 70:30, by mass.

The physiologically active composition contains, in addition to the "physiologically active substance" (A) and the "solvent including at least one polyhydric alcohol selected from the group consisting of glycerin, ethylene glycol, propylene glycol and 1,3-butylene glycol" (B), a polymer compound or a compound such as a metal chloride different from the physiologically active substance. A physiologically active composition containing the polymer compound or the compound such as a metal chloride can have an increased viscosity. A drug having a large molecular weight and a high solubility with respect to the solvent may work as a thickener by itself. However, when a drug has a low solubility with respect to the solvent or has a small molecular weight, the physiologically active composition may be required to further contain a polymer compound or a compound such as a metal chloride different from the physiologically active substance in order to increase the viscosity of the physiologically active composition.

The polymer compound is selected from polyethylene glycol, chondroitin sulfate, dextran, or croscarmellose sodium. In particular, when propylene glycol is used as the solvent for the physiologically active composition, the polymer compound is preferably polyethylene glycol or chondroitin sulfate, while when glycerin is used as the solvent, the polymer compound is preferably dextran, croscarmellose sodium, or chondroitin sulfate.

Examples of the metal chloride include sodium chloride, potassium chloride, magnesium chloride, potassium chloride, aluminum chloride, and zinc chloride. In particular, when glycerin and/or propylene glycol is used as the solvent for the physiologically active composition, the metal chloride is preferably magnesium chloride.

In addition, a physiologically active composition containing the metal chloride can suppress the reduction in the amount of a drug on the micro-needle and/or the substrate when the micro-needle device is stored for a long time. In particular, when propylene glycol is used as the solvent for the physiologically active composition, the metal chloride is preferably magnesium chloride. Accordingly, when propylene glycol is used as the solvent for the physiologically active composition, the physiologically active composition deposited on the micro-needles preferably contains at least one compound selected from hydroxypropyl cellulose, polyethylene glycol, chondroitin sulfate, hyaluronic acid, and magnesium chloride. When glycerin is used as the solvent for the physiologically active composition, the physiologically active composition deposited on the micro-needles preferably contains at least one compound selected from dextran, croscarmellose sodium, chondroitin sulfate, and magnesium chloride.

In addition to such a compound, the physiologically active composition may contain, as necessary, a solubilizing agent or an absorbefacient such as propylene carbonate, crotamiton, 1-menthol, peppermint oil, limonene, and diisopropyl adipate, and an efficacy auxiliary agent such as methyl salicylate, glycol salicylate, 1-menthol, thymol, peppermint oil, nonylic acid vanillylamide, and capsicum extract.

The physiologically active composition may further contain, as necessary, a stabilizer, an antioxidant, an emulsifier, a surfactant, salts, and the like. In the present invention, the surfactant may be either a nonionic surfactant or an ionic surfactant (a cation surfactant, an anionic surfactant, or an amphoteric surfactant). However, a nonionic surfactant generally used as a base material for pharmaceutical products is desirable from the viewpoint of safety. Specific examples of the surfactant include a sugar alcohol fatty acid ester such as a sucrose fatty acid ester, a sorbitan fatty acid ester, a glycerin fatty acid ester, a polyglycerol fatty acid ester, a propylene glycol fatty acid ester, a polyoxyethylene sorbitan fatty acid ester, a polyoxyethylene glycerin fatty acid ester, a polyethylene glycol fatty acid ester, a polyoxyethylene castor oil, and a polyoxyethylene hydrogenated castor oil.

Other known pharmaceutical auxiliary additives may be added to the physiologically active composition as long as such pharmaceutical auxiliary additives adversely affect the features of the solubility and the viscosity necessary for the coating of the physiologically active composition and the properties and the physical properties of the dried physiologically active composition.

The physiologically active composition is required to have a certain degree of viscosity so as not to drip, and specifically to have a viscosity of about 100 to 100,000 cps at room temperature (25°C). The viscosity of the physiologically active composition is more preferably 100 to 60,000 cps. A physiologically active composition having a viscosity within the range can be deposited in a desired amount at once without depending on a material of the micro-needles 3. Generally, a physiologically active composition having a higher viscosity is likely to be deposited in a larger amount, and a physiologically active composition having a viscosity of less than 0.600 Pa.s (600 cps) makes it difficult to deposit the minimum amount of a physiologically active substance onto the micro-needles 3. However, surprisingly, a physiologically active composition having a viscosity of 45.000 Pa.s (45000 cps) or more conversely reduces the amount of a physiologically active substance in the physiologically active composition 5 deposited on the micro-needles. From such a characteristics, a physiologically active composition having a viscosity of more than 45.000 Pa.s (45000 cps) or more cannot be expected to achieve the amount of the physiologically active substance in the deposited physiologically active composition 5 depending on the amount used of the physiologically active substance, resulting in an economic disadvantage. Therefore, the physiologically active composition particularly preferably has a viscosity of 0.600 to 45.000 Pa.s (600 to 45000 cps).

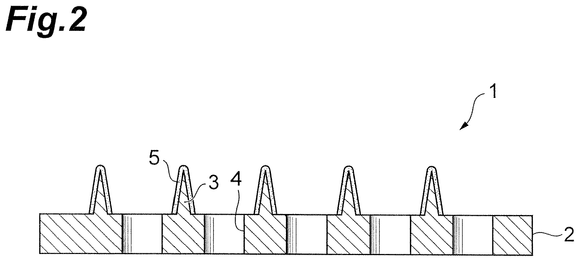

Fig. 2 is a sectional view taken along the line II-II in Fig. 1. As shown in Fig. 2, the micro-needle device 1 of the present invention includes the micro-needle substrate 2, the micro-needles 3 provided on the micro-needle substrate 2, and the physiologically active composition 5 deposited on the micro-needles 3 and/or the substrate. The deposited physiologically active composition 5 contains the "physiologically active substance" (A) and the "solvent including at least one polyhydric alcohol selected from the group consisting of glycerin, ethylene glycol, propylene glycol and 1,3-butylene glycol" (B), and is produced, for example, through a process shown in Figs. 3(a) to 3(c). Immediately after the production of the micro-needle device, the physiologically active composition contains the "solvent including at least one polyhydric alcohol selected from the group consisting of glycerin, ethylene glycol, propylene glycol, and 1,3-butylene glycol" contained in the physiologically active composition, at least one compound selected from polyethylene glycol, chondroitin sulfate, dextran, croscarmellose sodium and a metal chloride, and does not substantially contain water. However, the physiologically active composition may hold a solvent such as water due to a surrounding atmosphere during storage of the produced micro-needle device. The water content in this case is as mentioned above.

### [Examples]

Hereinafter, the present invention will be more specifically described with reference to examples of the present invention. The examples 1-3 are reference examples.

### (Reference Example 1) Solubility or Dispersibility Test to Solvent

Ten parts by mass of various physiologically active substances shown in Table 1 and 90 parts by mass of propylene glycol or glycerin were mixed for about 1 hour to give mixed solutions. Separately, as shown in Table 2, 43 parts by mass of physiologically active substance OVA (ovalbumin) and 57 parts by mass of triethanolamine, diethanolamine, or macrogol 400 were mixed in the same manner as the above to give a mixed solution. Then, the obtained mixed solutions were subjected to the visual evaluation of solubility or dispersibility of the physiologically active substances with respect to the solvents based on the criteria below. Each evaluation result is shown in Table 1 or Table 2.
a: A physiologically active substance was dissolved in a solvent (uniform liquid).
b: A physiologically active substance was dispersed in a solvent (dispersed liquid).
c: A physiologically active substance was not dissolved in a solvent and obvious aggregates were observed in a mixed solution (nonuniform liquid).

**[Table 1]**

| Physiologically active substance | Propylene glycol | Glycerin |
|---|---|---|
| Insulin | b | b |
| Human growth hormone | b | b |
| Gonadotropic hormone releasing hormone (LHRH) | a | a |
| Erythropoietin | b | b |
| Naltrexone | a | a |
| Cetrorelix acetate | a | b |
| Taltirelin | a | a |
| Nafarelin acetate | a | a |
| Octreotide acetate | a | a |
| Prostaglandin A1 | a | b |
| Alprostadil | b | b |

**[Table 2]**

| Physiologically active substance | triethanolamine | diethanolamine | macrogol 400 |
|---|---|---|---|
| OVA (ovalbumin) | b | b | b |

### (Reference Example 2) Relation Between Formulation of Physiologically Active Composition Containing Model Physiologically Active Substance (Octreotide Acetate) and Propylene Glycol or Glycen, and Viscosity and Amount of Physiologically Active Substance in Physiologically Active Composition Deposited on Micro-Needles

### <Setting Condition>

### (a) Micro-needles

* Material: polylactic acid, height: 500 µm, density: 625 needles/cm², preparation area on micro-needle substrate: 1 cm²/patch

### (b) Metal mask plate

* Pitch: 400 µm, mask thickness: 100 µm, opening: square shape (250 µm per side)

### (c) Environmental condition: room temperature (25°C)

### <Viscosity Measurement>

The octreotide acetate concentration and the propylene glycol or glycerin concentration were adjusted as shown in Table 3 and Table 4 to prepare a physiologically active composition. The viscosity of the obtained physiologically active composition was measured ten times with a micron sample viscometer (RHEOSENSE INC., Micron Sample-Viscometer/Rheometer-on-a-chip VROCTM), and the mean value was calculated to be shown in Table 2 and Table 3.

### <Determination of Amount of Octreotide Acetate in Physiologically Active Composition Deposited on Micro-Needles>

The octreotide acetate concentration and the propylene glycol or glycerin concentration were adjusted as shown in Table 3 and Table 4 to prepare a physiologically active composition. The physiologically active composition was deposited onto micro-needles in the manner shown in Figs. 3(a) to 3(c) above. The physiologically active composition was swept with a spatula to be filled in openings of a metal mask. Micro-needles (needles) were inserted into the filled openings and then pulled out of the openings. The physiologically active composition deposited on the micro-needles was extracted with purified water. The amount of octreotide acetate (deposition amount) on one patch (plate) of the micro-needle device was determined ten times by BCA method (octreotide standard), and the mean value was calculated to be shown in Table 3 and Table 4.

**[Table 3]**

| Octreotide acetate (% by weight) | Propylene glycol (% by weight) | Viscosity | Amount |
|---|---|---|---|
| | | Avg. (cps) | Avg. (µg/patch) |
| 20 | 80 | 200 | 4 |
| 40 | 60 | 1,600 | 36 |
| 50 | 50 | 5,400 | 121 |
| 60 | 40 | 15,000 | 243 |
| 70 | 30 | 45,000 | 237 |
| 80 | 20 | 133,000 | 97 |

**[Table 4]**

| Octreotide acetate (% by weight) | Glycerin (% by weight) | Viscosity | Amount |
|---|---|---|---|
| | | Avg. (cps) | Avg. (µg/patch) |
| 20 | 80 | 2,900 | 6 |
| 30 | 70 | 9,000 | 39 |
| 35 | 65 | 12,000 | 53 |
| 40 | 60 | 15,000 | 89 |
| 50 | 50 | 21,000 | 169 |
| 60 | 40 | 27,000 | 149 |

As shown in Table 3 and Table 4, it was revealed that the viscosity of the physiologically active composition increased with the increase of the amount of octreotide acetate in the physiologically active composition, while the amount of octreotide acetate in the physiologically active composition 5 deposited on the micro-needles increased with the increase of the viscosity to a certain viscosity but was shifted to reduction exceeding the certain viscosity.

In propylene glycol in Table 3, the amount of octreotide acetate was shifted to the reduction from a viscosity of 15,000 cps to 45,000 cps. This suggests that the suitable viscosity is from 200 cps to 45,000 cps and a viscosity more than the range is not preferred from the viewpoint of administration efficiency.

In glycerin in Table 4, the amount of octreotide acetate was shifted to the reduction from a viscosity of 21,000 cps to 27,000 cps. This suggests that the suitable viscosity is from 2,000 cps to 25,000 cps and a viscosity more than the range is not preferred from the viewpoint of administration efficiency.

### (Reference Example 3) Variation Measurement Test of Amount of Physiologically Active Substance in Physiologically Active Composition Deposited on Micro-Needles When Production Process of Micro-Needle Device Is Repeated

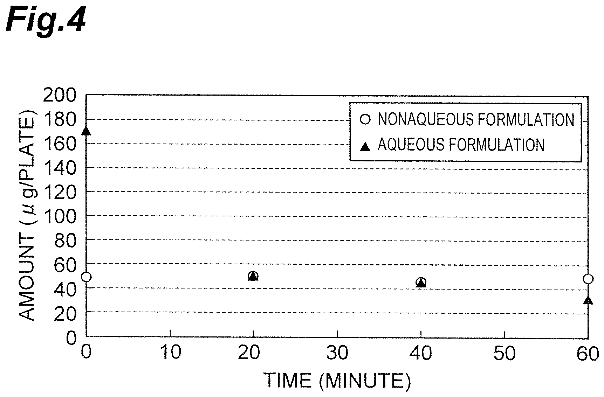

Into a PP (polypropylene) micro tube, 40 parts by mass of human plasma albumin (HSA) and 60 parts by mass of glycerin were added and dissolved to prepare a physiologically active composition in a nonaqueous formulation. As a control that was a physiologically active composition in an aqueous formulation, 40 parts by mass of human plasma albumin (HSA), 30 parts by mass of glycerin, and 30 parts by mass of water were mixed and the prepared mixed solution was dissolved to prepare a physiologically active composition. In order to produce a plurality of micro-needle devices, the filling and deposition process of each physiologically active composition was repeated in the same condition as in Reference Example 2. Immediately after the start of the deposition process and after 20 minutes, 40 minutes, and 60 minutes of the process, the amount of human plasma albumin (HSA) in the physiologically active composition deposited on the micro-needles of the obtained micro-needle device was determined in the same manner as in Reference Example 2. The obtained test results are shown in Fig. 4 as a graph.

In the nonaqueous formulation, the viscosity was constant over the production process and the variation in the amount of the physiologically active substance in the physiologically active composition deposited on the micro-needles was less observed. In contrast, in the aqueous formulation, it was ascertained that the viscosity increased in association with water vaporization with time, and the aqueous formulation also showed a tendency of remarkably reducing the amount of the physiologically active substance in the physiologically active composition with time.

### (Example 4) Viscosity Imparting Test to Physiologically Active Composition in Nonaqueous Formulation

With respect to each solvent of propylene glycol and glycerin, polymer compounds shown in Table 5 and Table 6 were added to prepare mixed solutions. Each concentration of the polymer compounds was designed in consideration of the molecular weight and the like. The prepared mixed solution was stirred (1,500 rpm, 12 hours, 25°C) with a stirrer and the solubility of the polymer compound was visually evaluated on the basis of the criteria below. Separately, the viscosity of the mixed solution or the solution after the stirring was determined with a micron sample viscometer at 25°C. The evaluation results of the viscosity and the solubility are shown in Tables 5 and 6.
a: Completely dissolved
b: Partly dissolved
c: Not dissolved

The viscosity and solubility test results of Dx 40 and Dx 70 that were added to glycerin as the solvent were obtained at a temperature of 80°C during stirring.

**[Table 5]**

| Solvent | Polymer compound | Additive concentration (% by mass) | Viscosity (mPa·s) | Solubility |
|---|---|---|---|---|
| Propylene glycol | without | - | 53 | - |
| | PEG 4000 | 5.0 | 94 | b |
| | Dx 40 | 2.5 | 44 | c |
| | Dx 70 | 2.5 | 58 | c |
| | Gelatin | 5.0 | 38 | c |
| | Protamine | 1.0 | 53 | c |
| | Carmellose Na 90 kDa | 2.5 | 57 | c |
| | Carmellose | 5.0 | 31 | c |
| | PVA 117 | 2.5 | 56 | c |
| | PVA 220 | 2.5 | 53 | c |
| | PVA 617 | 2.5 | 51 | c |
| | HPC-H | 5.0 | 15,717 | a |
| | HPC-M | 5.0 | 3,472 | a |
| | HPC-L | 5.0 | 2,181 | a |
| | Croscarmellose Na | 5.0 | 63 | c |
| | Starch | 5.0 | 59 | c |
| | HA | 2.5 | 70 | b |
| | Chondroitin sulfate | 2.5 | 71 | b |

**[Table 6]**

| Solvent | Polymer compound | Additive concentration (% by mass) | Viscosity (mPa·s) | Solubility |
|---|---|---|---|---|
| Glycerin | without | - | 1,419 | - |
| | PEG 4000 | 5.0 | 1,479 | c |
| | Dx 40 | 5.0 | 3,179 | a |
| | Dx 40 | 10.0 | 8,216 | a |
| | Dx 70 | 5.0 | 3,453 | a |
| | Gelatin | 5.0 | 1,507 | c |
| | Protamine | 0.5 | 1,442 | c |
| | Carmellose Na 90 kDa | 1.0 | 1,455 | c |
| | Carmellose | 5.0 | 1,241 | c |
| | PVA 117 | 2.5 | 1,349 | c |
| | PVA 220 | 2.5 | 1,374 | c |
| | PVA 617 | 2.5 | 1,334 | c |
| | HPC-H | 5.0 | 1,109 | c |
| | HPC-M | 5.0 | 1,314 | c |
| | HPC-L | 5.0 | 1,348 | c |
| | Croscarmellose Na | 5.0 | 1,751 | b |
| | Starch | 5.0 | 1,359 | c |
| | HA | 1.0 | 1,458 | c |
| | Chondroitin sulfate | 1.0 | 2,140 | b |

In Tables, PEG 4000 is polyethylene glycol having a weight average molecular weight of 4,000, Dx 40 and Dx 70 are dextrans having weight average molecular weights of about 40,000 and about 70,000, respectively, each of PVA 117, PVA 220, and PVA 617 is polyvinyl alcohol having a weight average molecular weight of about 75,000, HPC-H, HPC-M, and HPC-L are hydroxypropyl celluloses having weight average molecular weights of 250,000 to 400,000, 110,000 to 150,000, and 55,000 to 70,000, respectively, and HA is hyaluronic acid.

When the viscosity of a solution is increased by a small amount of a polymer compound, a physiologically active composition can be controlled to be a small thickness after the coating and drying. Thus, such a polymer compound is particularly preferred as a component in the physiologically active composition deposited on micro-needles. As shown in Table 5, hydroxypropyl cellulose has a high solubility with respect to propylene glycol, and the viscosity of the solution largely increased comparing with that before the addition of hydroxypropyl cellulose. A hydroxypropyl cellulose having a higher molecular weight was likely to increase the viscosity of a solution. From these results, HPC-H is expected to provide viscosity improvement effect even when the amount is small (low concentration). When the amount of HPC-H is reduced, a physiologically active substance can be added to the solution in a larger amount to prepare a physiologically active composition, thereby capable of further increasing the amount of the physiologically active substance on the micro-needles. Therefore, in Table 5, HPC-H is supposed to be the most suitable thickener with respect to propylene glycol.

PEG 4000, chondroitin sulfate, and HA were not completely dissolved in propylene glycol but were observed to provide viscosity improvement effect with respect to the solution or the mixed solution.

As shown in Table 6, dextran had a high solubility with respect to glycerin, and the viscosity of the solution largely increased comparing with that before the addition of dextran. A dextran having a higher molecular weight or a dextran having a higher concentration was likely to increase the viscosity of a solution. Croscarmellose sodium (Na) and chondroitin sulfate were not completely dissolved in glycerin but were observed to provide viscosity improvement effect with respect to the solution or the mixed solution.

From the results shown in Table 5 and Table 6, the polymer compounds suitable for the viscosity improvement were found with respect to each of propylene glycol and glycerin.

Viscosity Imparting Test to Physiologically Active Composition in Nonaqueous Formulation

### (Example 5)

With a stirrer, 7.3 parts by mass of propylene glycol, 0.7 part by mass of sodium hydroxide, and 2.0 parts by mass of magnesium chloride were stirred and mixed. The obtained mixed solution was further mixed with octreotide acetate at a mass ratio of 1:1 to give a physiologically active composition (50.0% by mass of octreotide acetate/3.5% by mass of sodium hydroxide/10.0% by mass of magnesium chloride/36.5% by mass of propylene glycol). The number of moles of sodium hydroxide added was equivalent to that of the acetate moiety in octreotide acetate.

The physiologically active composition was applied onto the tips of micro-needles similar to those in Reference Example 2 and dried. The height H of the physiologically active composition deposited on the micro-needles was measured under microscope observation. The evaluation result is shown in Table 7.

### (Comparative Example 1)

A physiologically active composition (50.0% by mass of octreotide acetate/3.5% by mass of sodium hydroxide/46.5% by mass of propylene glycol) was obtained in the same manner as in Example 5 except that magnesium chloride was not added and the same mass of propylene glycol was added instead. The physiologically active composition was applied onto micro-needles in the same manner as in Example 5 and the height H of the physiologically active composition deposited on the micro-needles was measured. The evaluation result is shown in Table 7.

### (Example 6)

With a stirrer, 8.434 parts by mass of glycerin, 0.233 part by mass of sodium hydroxide, and 1.333 parts by mass of magnesium chloride were stirred and mixed. The obtained mixed solution was further mixed with LHRH (luteinizing hormone-releasing hormone acetate) at a mass ratio of 3:1 to give a physiologically active composition (25.0% by mass of LHRH/1.75% by mass of sodium hydroxide/10.0% by mass of magnesium chloride/63.25% by mass of glycerin). The number of moles of sodium hydroxide added was equivalent to that of the acetate moiety in LHRH. The physiologically active composition was applied onto micro-needles in the same manner as in Example 5 and the height H of the physiologically active composition deposited on the micro-needles was measured. The evaluation result is shown in Table 7.

### (Comparative Example 2)

A physiologically active composition (25.0% by mass of LHRH/1.75% by mass of sodium hydroxide/73.25% by mass of glycerin) was obtained in the same manner as in Example 6 except that magnesium chloride was not added and the same mass of glycerin was added instead. The physiologically active composition was deposited onto micro-needles in the same manner as in Example 5 and the height H of the physiologically active composition deposited on the micro-needles was measured. The evaluation result is shown in Table 7.

**[Table 7]**

| | | Example 5 | Example 6 | Comp. Example 1 | Comp. Example 2 |
|---|---|---|---|---|---|
| Physiologically active substance (drug) | Octreotide acetate | 50.0 | - | 50.0 | - |
| | LHRH | - | 25.0 | - | 25.0 |
| Sodium hydroxide | | 3.5 | 1.75 | 3.5 | 1.75 |
| Magnesium chloride | | 10.0 | 10.0 | - | - |
| Solvent | Propylene glycol | 36.5 | - | 46.5 | - |
| | Glycerin | - | 63.25 | - | 73.25 |
| Height H (µm) of physiologically active composition deposited on micro-needles | | 130.2 | 137.4 | 302.6 | 229.4 |

As shown in Table 7, in Example 5 and Example 6, by the addition of magnesium chloride to the physiologically active composition, the physiologically active composition deposited on the micro-needles could be controlled to have a small thickness (to have a small height H). This is because the physiologically active composition obtained an improved viscosity to suppress dripping.

### Stability Test of Amount of Drug in Physiologically Active Composition Deposited on Micro-Needles

### (Example 7)

With a stirrer, 9.444 parts by mass of propylene glycol and 0.556 part by mass of magnesium chloride were stirred and mixed. The obtained mixed solution was further mixed with octreotide acetate at a mass ratio of 9:1 to give a physiologically active composition (10% by mass of octreotide acetate/5.0% by mass of magnesium chloride/85% by mass of propylene glycol).

Onto the whole area of micro-needles similar to Reference Example 2, 10 mg of the physiologically active composition was applied and dried at 50°C for 30 minutes to give a micro-needle device. Then, the obtained micro-needle device was placed in a package together with a preservative (PharmaKeep KD; manufactured by Mitsubishi Gas Chemical Company, Inc.) followed by sealing and the sealed micro-needle device was stored in a condition at 60°C for one week. Separately, another sealed micro-needle device was stored in a condition at 5°C for one week.

The amount of the physiologically active substance on the micro-needle device after storage was determined by high performance liquid chromatography (HPLC). Then, the residual ratio of the amount of the physiologically active substance on the micro-needles stored at 60°C with respect to the amount of the physiologically active substance on the micro-needles stored at 5°C was calculated as a percentage. The calculation result is shown in Table 8.

### (Comparative Example 3)

A physiologically active composition (10% by mass of octreotide acetate/90% by mass of propylene glycol) was obtained in the same manner as in Example 7 except that magnesium chloride was not added and the same mass of propylene glycol was added instead. A micro-needle device was obtained using the physiologically active composition in the same manner as in Example 7. The obtained micro-needle device was stored in the same manner as in Example 7 and the residual ratio of the physiologically active substance was calculated. The calculation result is shown in Table 8.

### (Example 8)

A micro-needle device was obtained in the same manner as in Example 7 except that the drug type was changed to LHRH, and the residual ratio of the physiologically active substance was calculated. The calculation result is shown in Table 8.

### (Comparative Example 4)

A micro-needle device was obtained in the same manner as in Comparative Example 3 except that the drug type was changed to LHRH, and the residual ratio of the physiologically active substance was calculated. The calculation result is shown in Table 8.

**[Table 8]**

| | | Example 7 | Example 8 | Comp. Example 3 | Comp. Example 4 |
|---|---|---|---|---|---|
| Physiologically active substance (drug) | Octreotide acetate | 10.0 | - | 10.0 | - |
| | LHRH | - | 10.0 | - | 10.0 |
| Magnesium chloride | | 5.0 | 5.0 | - | - |
| Propylene glycol | | 85.0 | 85.0 | 90.0 | 90.0 |
| Residual ratio (%) of physiologically active substance | | 94.0 | 99.3 | 79.5 | 98.0 |

As shown in Table 8, in Example 7 and Example 8, by the addition of magnesium chloride to the physiologically active composition, the residual ratio of the physiologically active substance could be maintained at a high value.

### Hairless Rat in Vivo Absorption Test of Lixisenatide

### (Example 9)

Into a tube, lixisenatide and propylene glycol were added so as to have a mass ratio of 50:50 and were mixed with a mixer to give a mixture as a physiologically active composition. The physiologically active composition was applied onto micro-needles using a mask plate having a thickness of 50 µm. The applied amount of the physiologically active substance was 12.2 µg/patch/head. Using a 0.4 J applicator having the coated micro-needle array, the physiologically active substance was administered to a hairless rat (the repeated number of tests: three times).

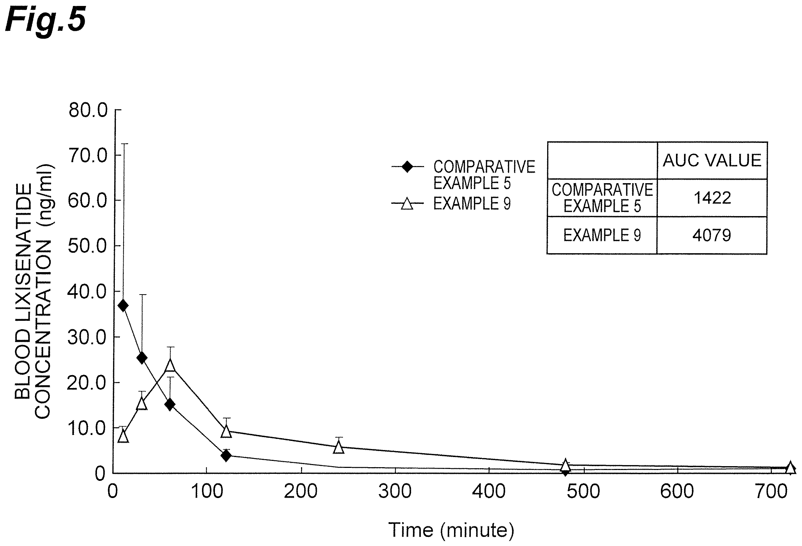

After 10 minutes, 30 minutes, 60 minutes, 120 minutes, 240 minutes, 480 minutes, and 720 minutes of the administration, 300 µL of blood specimen was collected from the jugular vein. The lixisenatide concentration in the blood was determined using Exendin-4 EIA Kit. The test result is shown in Fig. 5. The AUC value (area under the blood concentration-time curve) and the BA value (bioavailability) obtained from the graph in Fig. 5 are also shown in Table 9. The AUC value represents the area under the blood concentration-time curve in the range from 0 minute to 720 minutes after the administration in the graph in Fig. 5. The BA value represents the relative bioavailability value with respect to the subcutaneous administration.

### (Comparative Example 5)

Into a tube, lixisenatide and saline were added so as to have a mass ratio of 50:50 and were mixed with a mixer to give a mixture as a physiologically active composition. The physiologically active composition was subcutaneously administered to a hairless rat in a condition of 15.1 µg/300 µL/head. The lixisenatide concentration in the blood was determined in the same manner as in Example 9. The test result is shown in Fig. 5. The AUC value and the BA value are also shown in Table 9.

**[Table 9]**

| | Example 9 | Comparative Example 5 |
|---|---|---|
| AUC value (ng·min/mL) | 4,079 | 1,422 |
| BA value (%) | 355 | 100 |

### Hairless Rat in Vivo Absorption Test of β-Interferon

### (Example 10)

Into a tube, β-interferon and glycerin were added so as to have a mass ratio of 30:70 and were mixed with a mixer to give a mixture as a physiologically active composition. The physiologically active composition was applied onto micro-needles using a mask plate having a thickness of 100 µm. The applied amount of the physiologically active substance was 10.3 µg/patch/head. Using a 0.4 J applicator having the coated micro-needle array, the physiologically active substance was administered to a hairless rat (the repeated number of tests: three times).

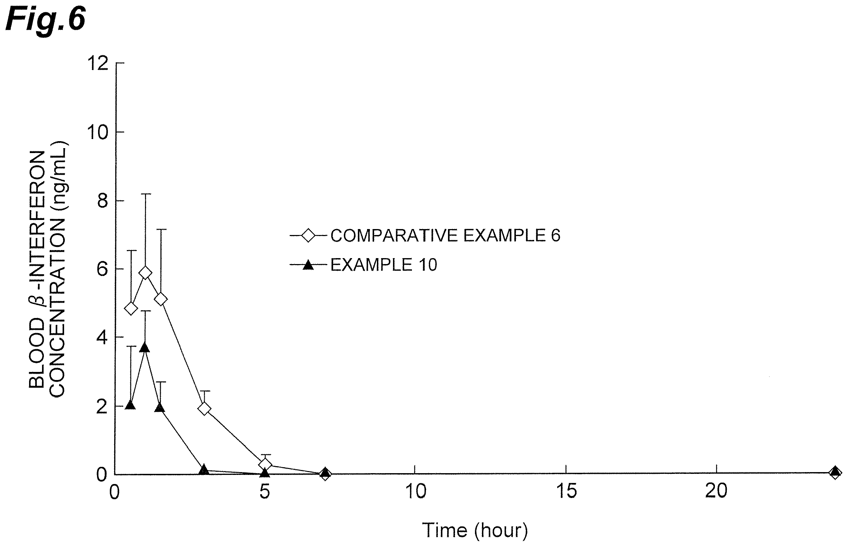

After 30 minutes, 60 minutes, 90 minutes, 180 minutes, 300 minutes, 420 minutes, and 1,440 minutes of the administration, 300 µL of blood specimen was collected from the jugular vein. The β-interferon concentration in the blood was determined using Exendin-4 EIA Kit. The test result is shown in Fig. 6.

### (Comparative Example 6)

Into a tube, β-interferon and saline were added so as to have a mass ratio of 50:50 and were mixed with a mixer to give a mixture as a physiologically active composition. The physiologically active composition was subcutaneously administered to a hairless rat in a condition of 10 µg/300 µL/head (the repeated number of tests: three times). Then, the β-interferon concentration in the blood was determined in the same manner as in Example 10. The test result is shown in Fig. 6.

### Industrial Applicability

According to the present invention, a micro-needle device in which the variation in the amount of a physiologically active substance in a physiologically active composition deposited on micro-needles is remarkably reduced can be obtained and the utility of the micro-needle is greatly increased. Therefore, the present invention has large industrial applicability.

### Reference Sings List

1... micro-needle device, 2... micro-needle substrate, 3... micro-needle, 5... physiologically active composition deposited on micro-needles, 10... physiologically active composition

## Claims

1. A micro-needle device comprising:
a substrate;
a micro-needle provided on the substrate; and
a physiologically active composition deposited on the micro-needle and/or the substrate,
wherein the physiologically active composition contains:
at least one polyhydric alcohol selected from glycerin, ethylene glycol, propylene glycol and 1,3-butylene glycol;
at least one compound selected from polyethylene glycol, chondroitin sulfate, dextran, croscarmellose sodium and a metal chloride; and
a physiologically active substance,
and contains substantially no water.

2. The micro-needle device according to claim 1, wherein the physiologically active composition is fixed on the micro-needle and/or the substrate.

3. A method for preparing a micro-needle device comprising the step of:
depositing a physiologically active composition containing:
at least one polyhydric alcohol selected from glycerin, ethylene glycol, propylene glycol and 1,3-butylene glycol;
at least one compound selected from polyethylene glycol, chondroitin sulfate, dextran, croscarmellose sodium and a metal chloride; and
a physiologically active substance,
and containing substantially no water
onto a micro-needle provided on a substrate.

4. The method for preparing a micro-needle device according to claim 3, wherein the mass ratio of the physiologically active substance and the polyhydric alcohol is 20:80 to 80:20.

5. The method for preparing a micro-needle device according to claim 3 or 4, wherein the physiologically active composition has a viscosity of 0.600 to 45.000 Pa·s (600 to 45000 cps) measured with a micron sample viscometer at a temperature of 25°C.

6. A method of stabilizing a deposition amount of a physiologically active composition, the method comprising the steps of:
storing a physiologically active composition containing:
at least one polyhydric alcohol selected from glycerin, ethylene glycol, propylene glycol and 1,3-butylene glycol;
at least one compound selected from polyethylene glycol, chondroitin sulfate, dextran, croscarmellose sodium and a metal chloride; and
a physiologically active substance,
and containing substantially no water
in a container from which the solvent is capable of volatilizing; and then depositing the physiologically active composition onto a micro-needle provided on a substrate to produce a micro-needle device.

## Patentansprüche

1. Mikronadelvorrichtung, umfassend:
ein Substrat,
eine Mikronadel, die auf dem Substrat bereitgestellt ist, und
eine physiologisch aktive Zusammensetzung, die auf der Mikronadel und/oder dem Substrat aufgetragen ist,
wobei die physiologisch aktive Zusammensetzung
mindestens einen mehrwertigen Alkohol, der aus Glycerin, Ethylenglykol, Propylenglykol und 1,3-Butylenglykol ausgewählt ist,
mindestens eine Verbindung, die aus Polyethylenglykol, Chondroitinsulfat, Dextran, Croscarmellose-Natrium und einem Metallchlorid ausgewählt ist, und
eine physiologisch aktive Substanz enthält
und im Wesentlichen kein Wasser enthält.

2. Mikronadelvorrichtung gemäß Anspruch 1, wobei die physiologisch aktive Zusammensetzung auf der Mikronadel und/oder dem Substrat fixiert ist.

3. Verfahren zum Anfertigen einer Mikronadelvorrichtung, umfassend den Schritt:
Auftragen einer physiologisch aktiven Zusammensetzung, die
mindestens einen mehrwertigen Alkohol, der aus Glycerin, Ethylenglykol, Propylenglykol und 1,3-Butylenglykol ausgewählt ist,
mindestens eine Verbindung, die aus Polyethylenglykol, Chondroitinsulfat, Dextran, Croscarmellose-Natrium und einem Metallchlorid ausgewählt ist, und
eine physiologisch aktive Substanz enthält
und im Wesentlichen kein Wasser enthält,
auf eine Mikronadel, die auf einem Substrat bereitgestellt ist.

4. Verfahren zum Anfertigen einer Mikronadelvorrichtung gemäß Anspruch 3, wobei das Massenverhältnis der physiologisch aktiven Substanz und des mehrwertigen Alkohols 20:80 bis 80:20 beträgt.

5. Verfahren zum Anfertigen einer Mikronadelvorrichtung gemäß Anspruch 3 oder 4, wobei die physiologisch aktive Zusammensetzung eine Viskosität von 0,600 bis 45,000 Pa·s (600 bis 45000 cps) aufweist, gemessen mit einem Micron-Sample-Viskosimeter bei einer Temperatur von 25 °C.

6. Verfahren zum Stabilisieren einer Ablagerungsmenge einer physiologisch aktiven Zusammensetzung, wobei das Verfahren die Schritte umfasst:
Lagern einer physiologisch aktiven Zusammensetzung, die
mindestens einen mehrwertigen Alkohol, der aus Glycerin, Ethylenglykol, Propylenglykol und 1,3-Butylenglykol ausgewählt ist,
mindestens eine Verbindung, die aus Polyethylenglykol, Chondroitinsulfat, Dextran, Croscarmellose-Natrium und einem Metallchlorid ausgewählt ist, und
eine physiologisch aktive Substanz enthält
und im Wesentlichen kein Wasser enthält,
in einem Behälter, aus dem das Lösungsmittel verdunsten kann und dann Auftragen der physiologisch aktiven Zusammensetzung auf eine Mikronadel, die auf einem Substrat bereitgestellt ist, um eine Mikronadelvorrichtung anzufertigen.

## Revendications

1. Dispositif à micro-aiguille comprenant :
un substrat ;
une micro-aiguille disposée sur le substrat ; et
une composition physiologiquement active déposée sur la micro-aiguille et/ou le substrat,
la composition physiologiquement active contenant :
au moins un poly(alcool hydrique) sélectionné parmi la glycérine, l'éthylène glycol, le propylène glycol et le 1,3-butylène glycol ;
au moins un composé sélectionné parmi le polyéthylène glycol, le sulfate de chondroïtine, le dextrane, la croscarmellose sodique et un chlorure métallique ; et
une substance physiologiquement active,
et qui ne contient sensiblement pas d'eau.

2. Dispositif à micro-aiguille selon la revendication 1, la composition physiologiquement active étant fixée sur la micro-aiguille et/ou le substrat.

3. Procédé de préparation d'un dispositif à micro-aiguille comprenant l'étape de :
dépôt d'une composition physiologiquement active contenant :
au moins un poly(alcool hydrique) sélectionné parmi la glycérine, l'éthylène glycol, le propylène glycol et le 1,3-butylène glycol ;
au moins un composé sélectionné parmi le polyéthylène glycol, le sulfate de chondroïtine, le dextrane, la croscarmellose sodique et un chlorure métallique ; et
une substance physiologiquement active,
et ne contenant sensiblement pas d'eau sur une micro-aiguille disposée sur un substrat.

4. Procédé de préparation d'un dispositif à micro-aiguille selon la revendication 3, le rapport en masse de la substance physiologiquement active et du poly(alcool hydrique) étant de 20:80 à 80:20.

5. Procédé de préparation d'un dispositif à micro-aiguille selon la revendication 3 ou 4, la composition physiologiquement active ayant une viscosité de 0,600 à 45 000 Pa•s (600 à 45 000 cps) mesurée à l'aide d'un viscosimètre d'échantillon de l'ordre du micron à une température de 25°C.

6. Procédé de stabilisation d'une quantité de dépôt d'une composition physiologiquement active, le procédé comprenant les étapes de :
stockage d'une composition physiologiquement active contenant :
au moins un poly(alcool hydrique) sélectionné parmi la glycérine, l'éthylène glycol, le propylène glycol et le 1,3-butylène glycol ;
au moins un composé sélectionné parmi le polyéthylène glycol, le sulfate de chondroïtine, le dextrane, la croscarmellose sodique et un chlorure métallique ; et
une substance physiologiquement active,
et ne contenant sensiblement pas d'eau dans un récipient à partir duquel le solvant est capable de se volatiliser ; et ensuite le dépôt de la composition physiologiquement active sur une micro-aiguille disposée sur un substrat pour produire un dispositif à micro-aiguille.
